(19) Europäisches Patentamt
European Patent Office
Office européen des brevets

(11) Veröffentlichungsnummer: **0 274 654**
**A2**

# (12) EUROPÄISCHE PATENTANMELDUNG

(21) Anmeldenummer: **87117995.8**

(22) Anmeldetag: **04.12.87**

(51) Int. Cl.⁴: **C07D 513/04** , C07D 471/04 , C07D 487/04 , A61K 31/53 , //(C07D513/04,275:00,253:00), (C07D471/04,253:00,221:00), (C07D487/04,253:00,231:00)

Patentansprüche für folgende Vertragsstaaten: ES + GR.

(30) Priorität: **12.12.86 GB 8629711**
**14.09.87 GB 8721564**

(43) Veröffentlichungstag der Anmeldung:
**20.07.88 Patentblatt 88/29**

(84) Benannte Vertragsstaaten:
**AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Attwood, Michael Richard**
**1A Benslow Lane**
**Hitchin Herts.(GB)**
Erfinder: **Crackett, Peter Hedley**
**56 Chilvers Bank**
**Baldock Herts.(GB)**
Erfinder: **Lawton, Geoffrey**
**109 Whitehill Road**
**Hitchin Herts.(GB)**

(74) Vertreter: **Lederer, Franz, Dr. et al**
**Van der Werth, Lederer & Riederer**
**Patentanwälte Lucile-Grahn-Strasse 22**
**D-8000 München 80(DE)**

(54) **1,2,3-Triazin-4(3H)-on-Derivate, Verfahren zu deren Herstellung sowie diese enthaltende Arzneimittel.**

(57) Verbindungen der Formel

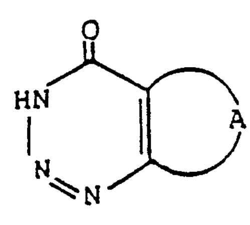

I



Xerox Copy Centre

worin A eine Gruppe der Formel

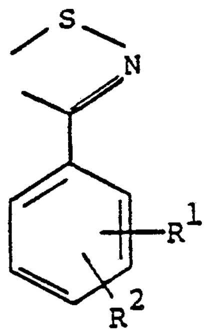

(a),

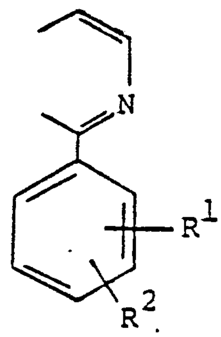

(b) , oder

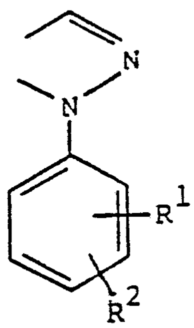

(c)

bedeutet, worin $R^1$ und $R^2$ je Wasserstoff, Halogen, Trifluormethyl, Nitro, Amino, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^3$ oder, wenn sie benachbart sind, zusammen eine Gruppe der Formel $-CH=CH-CH=CH-$oder $-CH_2-CH_2-O-$und $R^3$ Hydroxy-($C_1$-$C_4$-alkyl) oder vicinales Dihydroxy-($C_2$-$C_5$-alkyl) bedeuten,

und pharmazeutisch verwendbare Säureadditionssalze solcher Verbindungen der Formel I, worin $R^1$ und/oder $R^2$ Amino bedeuten, besitzen Xanthinoxidase hemmende Eigenschaften und können demnach als Arzneimittel, insbesondere bei der Bekämpfung bzw. Verhütung von Ischämie oder Gicht verwendet werden. Sie können nach bekannten Methoden hergestellt werden.

**1,2,3-Triazin-4(3H)-on-Derivate, Verfahren zu deren Herstellung sowie diese enthaltende Arzneimittel**

Die vorliegende Erfindung betrifft Triazinderivate, ein Verfahren zur deren Herstellung sowie Arzneimittel enthaltend diese Derivate.

Die erfindungsgemässen Triazinderivate sind Verbindungen der allgemeinen Formel

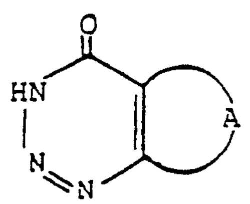

I

worin A eine Gruppe der Formel

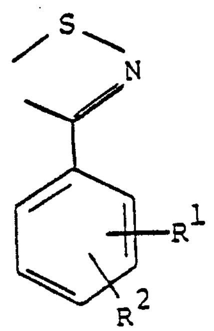

(a),

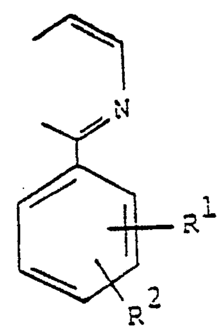

(b) oder

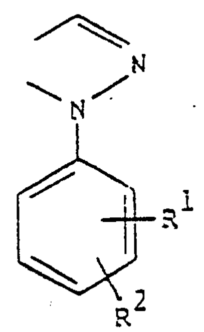

(c)

bedeutet, worin $R^1$ und $R^2$ je Wasserstoff, Halogen, Trifluormethyl, Nitro, Amino, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^3$ oder, wenn sie benachbart sind, zusammen eine Gruppe der Formel -CH = CH-CH = CH-oder $-CH_2-CH_2-O-$und $R^3$ Hydroxy-($C_1$-$C_4$-alkyl) oder vicinales Dihydroxy-($C_2$-$C_5$-alkyl) bedeuten,

und pharmazeutisch verwendbare Säureadditionssalze solcher Verbindungen der Formel I, worin $R^1$ und/oder $R^2$ Amino bedeuten.

Die in der vorliegenden Beschreibung verwendeten Ausdrücke "$C_1$-$C_4$-Alkyl", "$C_2$-$C_5$-Alkyl" und "$C_1$-$C_6$-Alkyl" betreffend geradkettige oder verzweigte Alkylreste mit soviel Kohlenstoffatomen wie angegeben, wie Methyl, Aethyl, n-Propyl, Isopropyl, n-Butyl, sek.Butyl, tert.Butyl, n-Pentyl, n-Hexyl und dergleichen. Der Ausdruck "$C_1$-$C_6$-Alkoxy" betrifft eine $C_1$-$C_6$-Alkylgruppe wie oben definiert, welche über ein Sauerstoffatom gebunden ist. Beispiele von $C_1$-$C_6$-Alkoxygruppen sind Methoxy, Aethoxy, n-Propoxy, Isopropoxy, n-Butoxy, Isobutoxy, tert.Butoxy und dergleichen. Der Ausdruck "$C_3$-$C_6$-Alkenyloxy" betrifft eine geradkettige oder verzweigte Alkenyloxygruppe mit 3-6 Kohlenstoffatomen, wie Allyloxy, Butenyloxy und dergleichen. Der

Ausdruck "$C_1$-$C_6$-Alkylthio" betrifft eine $C_1$-$C_6$-Alkylgruppe wie oben definiert, welche über ein Schwefelatom gebunden ist. Beispiele von $C_1$-$C_6$-Alkylthiogruppen sind Methylthio, Aethylthio, n-Propylthio, Isopropylthio, n-Butylthio und dergleichen. Der $C_1$-$C_6$-Alkanoylrest einer $C_1$-$C_6$-Alkanoylaminogruppe leitet sich von einer geradkettigen oder verzweigten Alkancarbonsäure mit 1-6 Kohlenstoffatomen ab, wie Formyl, Acetyl, Propionyl, Butyryl und dergleichen. Der Arylrest einer Aryloxy-, Aryl-($C_1$-$C_6$-alkyl)-, Aryl-($C_1$-$C_6$-alkoxy)-oder Aryl-($C_1$-$C_6$-alkoxy)carbonylaminogruppe ist Phenyl oder durch Halogen, Trifluormethyl, $C_1$-$C_6$-Alkyl, $C_1$-$C_6$-Alkoxy, Nitro und Cyan ein-oder mehrfach substituiertes Phenyl. Phenoxy, 4-Chlorphenoxy, 4-Tolyloxy und dergleichen sind Beispiele von Aryloxygruppen, Benzyl, 4-Chlorbenzyl, 4-Tolyl, 4-Methoxybenzyl, Phenäthyl und dergleichen sind Beispiele von Aryl-($C_1$-$C_6$-alkyl)gruppen und Benzyloxy, 4-Chlorbenzyloxy, 4-Tolyloxy, 4-Methoxybenzyloxy und dergleichen sind Beispiele von Aryl-($C_1$-$C_6$-alkoxy)gruppen. Beispiele von Gruppen der Formel $-O-CH_2-R^3$ sind 2-Hydroxyäthoxy, 3-Hydroxypropoxy und dergleichen, falls $R^3$ Hydroxy-($C_1$-$C_4$-alkyl) bedeutet, und 2,3-Dihydroxypropoxy, 3,4-Dihydroxybutoxy und dergleichen, falls $R^3$ vicinales Dihydroxy-($C_2$-$C_5$-alkyl) bedeutet. Der Ausdruck "Halogen" betrifft Fluor, Chlor, Brom und Jod.

Die Verbindungen der Formel I, worin $R^1$ und/oder $R^2$ Amino bedeuten, bilden pharmazeutisch verwendbare Salze mit Säuren. Beispiele solcher Salze sind Mineralsäuresalze, wie Hydrohalogenide, z.B. Hydrochloride, Hydrobromide und dergleichen, Sulfate, Phosphate, Nitrate und dergleichen, und Salze mit organischen Säuren, wie Acetate, Maleate, Fumarate, Tartrate, Citrate, Salicylate, Methansulfonate, p-Toluolsulfonate und dergleichen.

Es ist klar, dass die Verbindungen der Formel I in tautomeren Formen der Formeln

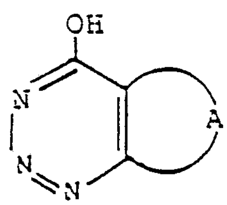

und

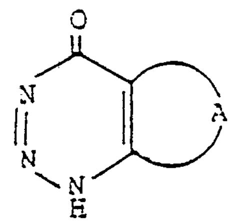

worin A die oben angegebene Bedeutung besitzt, existieren können, und dass solche tautomeren Formen ebenfalls Gegenstand der vorliegenden Erfindung sind.

Eine bevorzugte Klasse von Verbindungen der Formel I sind solche, worin A die Gruppe (a) bedeutet. In solchen Verbindungen bedeutet $R^1$ vorzugsweise Wasserstoff, Halogen, Trifluormethyl oder Cyan und $R^2$ vorzugsweise Wasserstoff, $C_1$-$C_6$-Alkoxy, Aryl-($C_1$-$C_6$-alkoxy) oder eine Gruppe der Formel $-O-CH_2-R^3$ worin $R^3$ vicinales Dihydroxy-($C_2$-$C_5$-alkyl) bedeutet, mit der Massgabe, dass mindestens einer von $R^1$ und $R^2$ von Wasserstoff verschieden ist.

Die am meisten bevorzugten Verbindungen der Formel I, worin A die Gruppe (a) bedeutet, sind:

7-(3-Trifluormethyl-4-methoxyphenyl)isothiazolo[4,5-d]-1,2,3-triazin-4(3H)-on,
7-(3-Chlor-4-methoxyphenyl)isothiazolo[4,5-d]-1,2,3-triazin-4(3H)-on,
7-(3-Fluor-4-methoxyphenyl)isothiazolo[4,5-d]-1,2,3-triazin-4(3H)-on,
7-(3-Trifluormethylphenyl)isothiazolo[4,5-d]-1,2,3-triazin-4(3H)-on,
7-(4-Isopropoxyphenyl)isothiazolo[4,5-d]-1,2,3-triazin-4(3H)-on,
7-(4-Benzyloxyphenyl)isothiazolo[4,5-d]-1,2,3-triazin-4(3H)-on,
7-(3-Cyan-4-methoxyphenyl)isothiazolo[4,5-d]-1,2,3-triazin-4(3H)-on und
7-[3-Cyan-4-(2,3-dihydroxypropoxy)phenyl]isothiazolo[4,5-d]-1,2,3-triazin-4(3H)-on.

Eine andere bevorzugte Klasse von Verbindungen der Formel I sind solche, worin A die Gruppe (c) bedeutet. In solchen Verbindungen bedeutet $R^1$ vorzugsweise Wasserstoff und $R^2$ vorzugsweise $C_1$-$C_6$-Alkoxy.

Die bevorzugteste Verbindung der Formel I, worin A die Gruppe (c) bedeutet, ist das 7-(4-Methoxyphenyl)-7H-pyrazolo[3,4-d] -1,2,3-triazin-4(3H)-on.

Beispiele weiterer interessanter Verbindungen der Formel I sind:

7-Phenylisothiazolo[4,5-d]-1,2,3-triazin-4(3H)-on,
7-(3-Aethylphenyl)isothiazolo[4,5-d]-1,2,3-triazin-4(3H)-on,
7-(3-Methylphenyl)isothiazolo[4,5-d]-1,2,3-triazin-4(3H)-on,
7-(3-Chlorphenyl)isothiazolo[4,5-d]-1,2,3-triazin-4(3H)-on,
7-(3-Bromphenyl)isothiazolo[4,5-d]-1,2,3-triazin-4(3H)-on,

7-(3-Cyanphenyl)isothiazolo[4,5-d]-1,2,3-triazin-4(3H)-on,
7-(3-Methoxyphenyl)isothiazolo[4,5-d]-1,2,3-triazin-4(3H)-on,
7-(4-Methoxyphenyl)isothiazolo[4,5-d]-1,2,3-triazin-4(3H)-on,
7-(3-Fluorphenyl)isothiazolo[4,5-d]-1,2,3-triazin-4(3H)-on,
7-(4-Acetamidophenyl)isothiazolo[4,5-d]-1,2,3-triazin-4(3H)-on,
7-(4-Benzyloxyformamidophenyl)isothiazolo[4,5-d]-1,2,3-triazin-4(3H)-on,
7-(4-Aminophenyl)isothiazolo[4,5-d]-1,2,3-triazin-4(3H)-on,
7-(4-Nitrophenyl)isothiazolo[4,5-d]-1,2,3-triazin-4(3H)-on,
7-(4-Chlorphenyl)isothiazolo[4,5-d]-1,2,3-triazin-4(3H)-on,
7-(4-Bromphenyl)isothiazolo[4,5-d]-1,2,3-triazin-4(3H)-on,
7-(4-Phenoxyphenyl)isothiazolo[4,5-d]-1,2,3-triazin-4(3H)-on,
7-(2-Naphthyl)isothiazolo[4,5-d]-1,2,3-triazin-4(3H)-on,
7-(2,3,-Dihydro-5-benzofuranyl)isothiazolo[4,5-d]-1,2,3-triazin-4(3H)-on,
7-(3,5-Dimethylphenyl)isothiazolo[4,5-d]-1,2,3-triazin-4(3H)-on,
7-(4-Benzylphenyl)isothiazolo[4,5-d]-1,2,3-triazin-4(3H)-on,
7-(3-Isopropylphenyl)isothiazolo[4,5-d]-1,2,3-triazin-4(3H)-on,
7-[3,5-bis(Trifluormethyl)phenyl]isothiazolo[4,5-d]-1,2,3-triazin-4(3H)-on,
7-(3-Trifluormethyl-4-benzyloxyphenyl)isothiazolo[4,5-d]-1,2,3-triazin-4(3H)-on,
8-Phenylpyrido[3,4-d]-1,2,3-triazin-4(3H)-on,
8-(3-Trifluormethylphenyl)pyrido[3,4-d]-1,2,3-triazin-4(3H)-on,
7-Phenyl-7H-pyrazolo[3,4-d]-1,2,3-triazin-4(3H)-on,
7-(3-Trifluormethylphenyl)-7H-pyrazolo[3,4-d]-1,2,3-triazin-4(3H)-on,
7-(3-Chlorphenyl)-7H-pyrazolo[3,4-d]-1,2,3-triazin-4(3H)-on,
7-(4-Aethylphenyl)-7H-pyrazolo[3,4-d]-1,2,3-triazin-4(3H)-on,
7-(4-Methylthiophenyl)-7H-pyrazolo[3,4-d]-1,2,3-triazin-4(3H)-on und
7-(3-Chlor-4-methoxyphenyl)-7H-pyrazolo[3,4-d]-1,2,3-triazin-4(3H)-on.

Weitere interessante Verbindungen der Formel I sind:

7-(4-Aethylphenyl)isothiazolo[4,5-d]-1,2,3-triazin-4(3H)-on,
7-(4-Isobutylphenyl)isothiazolo[4,5-d]-1,2,3-triazin-4(3H)-on,
7-(4-Allyloxy-3-cyanphenyl)isothiazolo[4,5-d]-1,2,3-triazin-4(3H)-on,
7-[3-Cyan-4-(2-hydroxyäthoxy)phenyl]isothiazolo[4,5-d]-1,2,3-triazin-4(3H)-on,
7-[3-Cyan-4-(3-hydroxypropoxy)phenyl]isothiazolo[4,5-d]-1,2,3-triazin-4(3H)-on,
7-(3-Cyan-4-methylthiophenyl)isothiazolo[4,5-d]-1,2,3-triazin-4(3H)-on,
8-(4-Methoxyphenyl)pyrido[3,4-d]-1,2,3-triazin-4(3H)-on,
8-(4-Benzyloxyphenyl)pyrido[3,4-d]-1,2,3-triazin-4(3H)-on,
8-(3-Fluor-4-methoxyphenyl)pyrido[3,4-d]-1,2,3-triazin-4(3H)-on,
8-(3-Chlor-4-methoxyphenyl)pyrido[3,4-d]-1,2,3-triazin-4(3H)-on,
8-(4-Methoxy-3-trifluormethylphenyl)pyrido[3,4-d]-1,2,3-triazin-4(3H)-on und
8-(4-Benzyloxy-3-trifluormethylphenyl)pyrido[3,4-d]-1,2,3-triazin-4(3H)-on.

Die Verbindungen der Formel I und die pharmazeutisch verwendbaren Salze solcher Verbindungen der Formel I, worin $R^1$ und/oder $R^2$ Amino bedeuten, können erfindungsgemäss dadurch hergestellt werden, dass man

a) zur Herstellung einer Verbindung der Formel I, worin $R^1$ und $R^2$ je Wasserstoff, Halogen, Trifluormethyl, Nitro, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^3$ oder, wenn sie benachbart sind, zusammen eine Gruppe der Formel $-CH=CH-CH=CH-$oder $-CH_2-CH_2-O-$und $R^3$ Hydroxy-($C_1$-$C_4$-alkyl) bedeuten, eine Verbindung der allgemeinen Formel

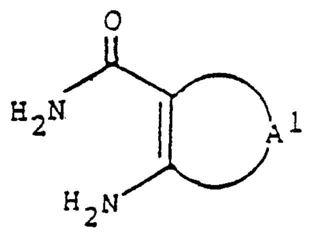

II

worin $A^1$ eine Gruppe der Formel

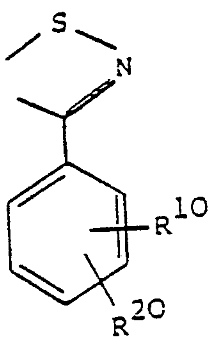

(a'),

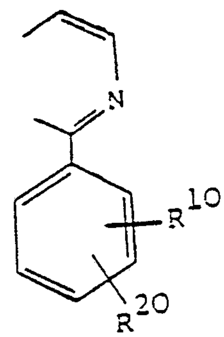

(b') oder

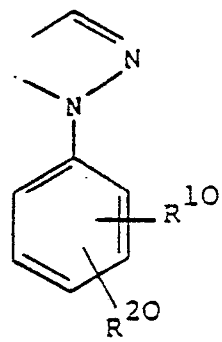

(c')

bedeutet, worin $R^{10}$ und $R^{20}$ je Wasserstoff, Halogen, Trifluormethyl, Nitro, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^{30}$ oder, wenn sie benachbart sind, zusammen eine Gruppe der Formel $-CH=CH-CH=CH-$oder $-CH_2-CH_2-O-$und $R^{30}$ Hydroxy-($C_1$-$C_4$-alkyl) bedeuten,

mit salpetriger Säure umsetzt, oder

b) zur Herstellung einer Verbindung der Formel I, worin $R^1$ und/oder $R^2$ Amino bedeuten, die Aryl-($C_1$-$C_6$-alkoxy)carbonylaminogruppe(n) in einer Verbindung der Formel I, worin $R^1$ und/oder $R^2$ Aryl-($C_1$-$C_6$-alkoxy)carbonylamino bedeutet, spaltet, oder

c) zur Herstellung einer Verbindung der Formel I, worin $R^1$ und/oder $R^2$ $C_1$-$C_6$-Alkanoylamino bedeuten, eine Verbindung der Formel I, worin $R^1$ und/oder $R^2$ Amino bedeuten, entsprechend acyliert, oder

d) zur Herstellung einer Verbindung der Formel I, worin $R^1$ und/oder $R^2$ eine Gruppe der Formel $-O-CH_2-R^3$ bedeuten, worin $R^3$ Hydroxy-($C_1$-$C_4$-alkyl) bedeutet, eine Verbindung der Formel I, worin $R^1$ und/oder $R^2$ $C_3$-$C_6$-Alkenyloxy bedeuten, mit Ozon umsetzt und das Reaktionsprodukt mit einem komplexen Metallhydrid reduziert, oder

e) zur Herstellung einer Verbindung der Formel I, worin $R^1$ und/oder $R^2$ eine Gruppe der Formel $-O-CH_2-R^3$ bedeuten, worin $R^3$ vicinales Dihydroxy-($C_2$-$C_5$-alkyl) bedeutet, eine Verbindung der Formel I, worin $R^1$ und/oder $R^2$ $C_3$-$C_6$-Alkenyloxy bedeuten, mit Osmiumtetroxid umsetzt, und

f) erwünschtenfalls, eine erhaltene Verbindung der Formel I, worin $R^1$ und/oder $R^2$ Amino bedeuten, in ein pharmazeutisch verwendbares Säureadditionssalz überführt.

Die Umsetzung einer Verbindung der Formel II mit salpetriger Säure gemäss Verfahrensvariante a) kann in bekannter Weise durchgeführt werden. So kann beispielsweise eine Verbindung der Formel II mit einer starken anorganischen Säure, zweckmässigerweise einer Halogenwasserstoffsäure, wie Chlorwasserstoffsäure, und einem Alkalimetallnitrit, zweckmässigerweise Natriumnitrit, in einem wässrigen Medium bei

tiefer Temperatur, z.B. ungefähr 0°C, umgesetzt werden, wobei die salpetrige Säure in situ gebildet wird. Erwünschtenfalls kann das wässrige Medium ein inertes, mit Wasser mischbares organisches Lösungsmittel enthalten, wie eine $C_2$-$C_6$-Alkancarbonsäure, beispielsweise Essigsäure, ein $C_1$-$C_6$-Alkanol, beispielsweise Aethanol, N,N-Dimethylformamid, Dimethylsulfoxid, N-Methylpyrrolidon und dergleichen. Gemäss einer anderen Ausführungsform kann eine Verbindung der Formel II mit Isoamylnitrit in Gegenwart eines inerten organischen Lösungsmittels, welches mit Wasser nicht mischbar ist, beispielsweise einem halogenierten Kohlenwasserstoff, wie Chloroform, erhitzt werden, vorzugsweise auf die Rückflusstemperatur.

Die Spaltung der Aryl-(nieder-alkoxy)carbonylaminogruppe(n), vorzugsweise der Benzyloxycarbonylaminogruppe(n), gemäss Verfahrensvariante b) kann ebenfalls in bekannter Weise durchgeführt werden. Vorzugsweise erfolgt die Spaltung durch Behandlung mit einer Säure, zweckmässigerweise einer halogenierten $C_2$-$C_6$-Alkancarbonsäure, vorzugsweise Trifluoressigsäure, vorteilhafterweise bei einer erhöhten Temperatur, z.B. bei der Rückflusstemperatur, obwohl jede andere geeignete Säure, beispielsweise Bromwasserstoff in Eisessig oder Chlorwasserstoff in Aethylacetat, ebenfalls verwendet werden kann. Die Spaltung kann auch durch Hydrieren in Gegenwart eines geeigneten Katalysators wie Palladium auf Kohle, zweckmässigerweise in einem inerten organischen Lösungsmittel, wie einem $C_1$-$C_6$Alkanol, beispielsweise Methanol, Aethanol und dergleichen, bei ungefähr Raumtemperatur und Atmosphärendruck erfolgen. Die Spaltung mittels katalytischer Hydrierung ist jedoch dann nicht bevorzugt, wenn A in der Verbindung der Formel I die Gruppe (a) bedeutet.

Auch die Acylierung einer Verbindung der Formel I, worin $R^1$ und/oder $R^2$ Amino bedeuten, gemäss Verfahrensvariante c) kann in bekannter Weise erfolgen. Zweckmässigerweise wird die Acylierung unter Verwendung eines geeigneten Alkancarbonsäureanhydrids, wie Essigsäureanhydrid, bei erhöhter Temperatur, beispielsweise ungefähr 100°C, durchgeführt. Die Acylierung kann jedoch auch unter Verwendung anderer reaktiver Derivate geeigneter Alkancarbonsäuren nach bekannten Methoden durchgeführt werden, beispielsweise mit Alkancarbonsäurehalogeniden, wie Chloriden.

Die Umsetzung einer Verbindung der Formel I, worin $R^1$ und/oder $R^2$ $C_3$-$C_6$-Alkenyloxy bedeuten, mit Ozon und Reduktion des erhaltenen Produktes mit einem komplexen Metallhydrid gemäss Verfahrensvariante d) kann ebenfalls nach bekannten Methoden durchgeführt werden. Beispielsweise erfolgt die Umsetzung mit Ozon zweckmässigerweise so, dass man bei ungefähr Raumtemperatur Ozon durch eine Lösung des Ausgangsmaterials der Formel I in einem inerten organischen Lösungsmittel, wie einem $C_1$-$C_6$-Alkanol, z.B. Methanol, Aethanol und dergleichen, einem cyclischen Aether, z.B. Tetrahydrofuran und dergleichen, oder einem Gemisch davon, durchleitet. Das Reaktionsprodukt wird danach zweckmässigerweise ohne Isolierung bei vorzugsweise etwa Raumtemperatur mit einem komplexen Metallhydrid, wie einem Alkalimetallborhydrid, z.B. Natriumborhydrid, Lithiumaluminiumhydrid, Diisobutylaluminiumhydrid oder dergleichen, reduziert.

Bekannte Methoden können auch zur Umsetzung einer Verbindung der Formel I, worin $R^1$ und/oder $R^2$ $C_3$-$C_6$-Alkenyloxy bedeuten, mit Osmiumtetroxid gemäss Verfahrensvariante e) verwendet werden. So kann die Umsetzung mit Osmiumtetroxid in einem mit Wasser mischbaren inerten organischen Lösungsmittel, z.B. Dimethylformamid, zweckmässigerweise bei ungefähr Raumtemperatur durchgeführt werden. Erwünschtenfalls kann die Umsetzung in Gegenwart eines Reagens erfolgen, wie N-Methylmorpholin-N-oxid, welches die während der Reaktion gebildeten Osmiumderivate zu Oxmiumtetroxid oxidiert. Nach beendeter Umsetzung wird das Reaktionsgemisch zweckmässigerweise mit einem Alkalimetalldithionit, vorzugsweise Natriumdithionit, zweckdienlich in Form einer wässrigen Lösung und bei etwa Raumtemperatur, umgesetzt, um die vorhandenen Osmiumsalze zu einer Form zu reduzieren, in welcher sie leicht vom erwünschten Produkt der Formel I abgetrennt werden können.

Die Ueberführung einer Verbindung der Formel I, worin $R^1$ und/oder $R^2$ Amino bedeuten, in ein pharmazeutisch verwendbares Säureadditionssalz gemäss Verfahrensvariante f) kann in bekannter Weise erfolgen. Beispielsweise wird das pharmazeutisch verwendbare Säureadditionssalz so gebildet, dass man eine Verbindung der Formel I mit einer Mineralsäure, z.B. einer Halogenwasserstoffsäure, wie Chlorwasserstoffsäure, Bromwasserstoffsäure und dergleichen, Schwefelsäure, Salpetersäure, Phosphorsäure und dergleichen, oder einer organischen Säure, wie Essigsäure, Maleinsäure, Fumarsäure, Weinsäure, Zitronensäure, Salicylsäure, Methansulfonsäure, p-Toluolsulfonsäure und dergleichen, umsetzt.

Die als Ausgangsstoffe eingesetzten Verbindungen der Formel II sind bekannt oder Analoge bekannter Verbindungen und können in ähnlicher Weise wie die bekannten Verbindungen hergestellt werden. Bezüglich der genauen Reaktionsbedingungen wird auf die nachstehenden Beispiele hingewiesen.

Die Verbindungen der Formel I und die pharmazeutisch verwendbaren Säureadditionssalze solcher Verbindungen, worin $R^1$ und/oder $R^2$ Amino bedeuten, besitzen wertvolle pharmakodynamische Eigenschaften. Insbesondere besitzen sie Xanthinoxidase hemmende Wirkung und können demnach bei der Bekämpfung bzw. Verhütung von Ischämie, welche myocardialen, cerebralen, renalen und/oder intestinalen

Ursprungs sein kann, oder Gicht verwendet werden.

Die Xanthinoxidase hemmende Wirkung der Verbindungen der Formel I kann im folgenden Test gezeigt werden.

Xanthinoxidase wird aus Rattenleber gemäss der von E. Della Corte und F. Stirpe in Biochem. J. 117, 97 (1970) beschriebenen Methode gewonnen und mindestens 24 Stunden vor Gebrauch altern gelassen. Lösungen von 3 ml 0,1M wässrigem Tris-hydrochloridpuffer (pH 8,1), enthaltend $10^{5}$ molares Xanthin werden mit 200 µl Xanthinoxidase in 0,1M wässrigem Tris-hydrochloridpuffer (pH 8,1), gelöst und bei 30°C in Gegenwart und in Abwesenheit einer Testverbindung inkubiert, wobei die Bildung von Harnsäure aus Xanthin durch Messen der Lichtabsorption bei 293 nm überwacht wird. Der $IC_{50}$-Wert, d.h. diejenige Konzentration einer Testverbindung, welche die Xanthinoxidase-katalysierte Oxidation von Xanthin zu Harnsäure um 50% hemmt, wird dann bestimmt.

Die nachfolgende Tabelle enthält die Resultate, welche mit repräsentativen Vertretern der Verbindungen der Formel I als Testverbindungen erhalten wurden:

## Tabelle

| Verbindung der Formel I | $IC_{50}$ (nMol) |
|---|---|
| 7-(3-Trifluormethyl-4-methoxyphenyl)-isothiazolo[4,5-d]-1,2,3-triazin-4(3H)-on | 4 |
| 7-(3-Chlor-4-methoxyphenyl)isothiazolo-[4,5-d]-1,2,3-triazin-4(3H)-on | 4 |
| 7-(3-Fluor-4-methoxyphenyl)isothiazolo-[4,5-d]-1,2,3-triazin-4(3H)-on | 8 |
| 7-(4-Acetamidophenyl)isothiazolo[4,5-d]--1,2,3-triazin-4(3H)-on | 7 |
| 8-(3-Trifluormethylphenyl)pyrido[3,4-d]--1,2,3-triazin-4(3H)-on | 48 |
| 7-(3-Chlor-4-methoxyphenyl)-7H-pyrazolo-[3,4-d]-1,2,3-triazin-4(3H)-on | 6 |
| 7-(4-Methylthiophenyl)-7H-pyrazolo[3,4-d]--1,2,3-triazin-4(3H)-on | 18 |
| 7-(3-Trifluormethylphenyl)-7H-pyrazolo-[3,4-d]-1,2,3-triazin-4(3H)-on | 35 |

Die Verbindungen der Formel I und die pharmazeutisch verwendbaren Säureadditionssalze solcher Verbindungen, worin $R^1$ und/oder $R^2$ Amino bedeuten, können als Arzneimittel verwendet werden, z.B. in Form pharmazeutischer Präparate. Die pharmazeutischen Präparate können oral, beispielsweise in Form von Tabletten, Lacktabletten, Dragées, Hart-und Weichgelatinekapseln, Lösungen, Emulsionen oder Suspensionen verabreicht werden. Sie können aber auch rektal, z.B. in Form von Suppositorien, oder parenteral, z.B. in Form von Injektionslösungen, verabreicht werden.

Zur Herstellung der pharmazeutischen Präparate werden die Verbindungen der Formel I und die pharmazeutisch verwendbaren Säureadditionssalze solcher Verbindungen, worin $R^1$ und/oder $R^2$ Amino bedeuten, mit pharmazeutisch inerten, anorganischen oder organischen Excipientien verarbeitet. Als solche

Excipientien, welche für Tabletten, Lacktabletten, Dragées und Hartgelatinekapseln verwendet werden können, eignen sich beispielsweise Lactose, Maisstärke oder Derivate davon, Talk, Stearinsäure oder deren Salze. Für Weichgelatinekapseln eignen sich als Excipientien z.B. vegetabile Oele, Wachse, Fette, halbfeste und flüssige Polyole etc. Wasser, Polyole, Saccharose, Invertzucker, Glucose etc. sind Beispiele geeigneter Excipientien zur Herstellung von Lösungen und Sirupen. Geeignete Excipientien für Injektionslösungen sind z.B. Wasser, Alkohole, Polyole, Glycerin, vegetabile Oele etc. Natürliche oder gehärtete Oele, Wachse, Fette, halbflüssige oder flüssige Polyole etc. sind Beispiele geeigneter Excipientien für Suppositorien.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der Formel I und die pharmazeutisch verwendbaren Salze solcher Verbindungen, worin $R^1$ und/oder $R^2$ Amino bedeuten, bei der Bekämpfung bzw. Verhütung von Krankheiten, insbesondere von Ischämie oder Gicht verwenden. Die Dosierung der Verbindungen der Formel I kann innerhalb weiter Grenzen variieren und ist natürlich in jedem einzelnen Fall den individuellen Gegebenheiten anzupassen. Im allgemeinen dürfte bei oraler Verabreichung an Erwachsene eine Tagesdosis von etwa 5 mg bis etwa 500 mg angemessen sein, wobei aber die soeben angegebene obere Grenze auch überschritten werden kann, wenn sich dies als angezeigt erweisen sollte. Die Tagesdosis kann als Einzeldosis oder in verschiedene Dosen unterteilt verab reicht werden.

Die folgenden Beispiele veranschaulichen die vorliegende Erfindung.

Beispiel 1

219 mg 4-Amino-3-phenyl-5-isothiazolcarboxamid in 7,2 ml Eisessig und 4 ml konz. Salzsäure werden während der Zugabe einer Lösung von 80 mg Natriumnitrit in 1,2 ml Wasser bei 0°C gerührt. Das Reaktionsgemisch wird über Nacht unterhalb Raumtemperatur gehalten, und das ausgefallene Produkt abfiltriert und aus Methanol umkristallisiert, wobei man 164 mg 7-Phenylisothiazolo[4,5-d]-1,2,3-triazin-4(3H)-on erhält. Eine aus wässrigem Methanol umkristallisierte Probe schmilzt bei 179-180°C (Zers.).

Beispiel 2

496 mg 4-Amino-3-(3-äthylphenyl)-5-isothiazolcarboxamid in 7 ml Eisessig, 4 ml konz. Salzsäure und 4 ml N-Methylpyrrolidon werden während der Zugabe von 165 mg Natriumnitrit in 1 ml Wasser bei 0°C gerührt. Das Reaktionsgemisch wird während 1,5 Stunden bei 0°C gehalten, danach abfiltriert, und der feste Rückstand aus Aethanol umkristallisiert, wobei man 400 mg 7-(3-Aethylphenyl)isothiazolo[4,5-d]-1,2,3-triazin-4(3H)-on erhält, Smp. 156-157°C (Zers.).

Beispiel 3

1 g 4-Amino-3-(4-methoxyphenyl)-5-isothiazolcarboxamid in 10 ml Eisessig und 3 ml konz. Salzsäure wird während der Zugabe einer Lösung von 416 mg Natriumnitrit in 5 ml Wasser bei 0°C gerührt. Das Kühlbad wird entfernt, das Reaktionsgemisch 1 Stunde gerührt, und das ausgefallene Produkt abfiltriert. Umkristallisation aus wässrigem Dimethyl formamid liefert 820 mg 7-(4-Methoxyphenyl)isothiazolo[4,5-d]-1,2,3-triazin-4(3H)-on als weisslichen Festkörper, Smp. 213°C (Zers.).

Beispiel 4

Die folgenden Verbindungen wurden in analoger Weise wie in den Beispielen 1-3 beschrieben hergestellt:

7-(3-Methylphenyl)isothiazolo[4,5-d]-1,2,3-triazin-4(3H)-on, Smp. 181-183°C (Zers.);
7-(3-Chlorphenyl)isothiazolo[4,5-d]-1,2,3-triazin-4(3H)-on, Smp. 190°C (Zers.);
7-(3-Bromphenyl)isothiazolo[4,5-d]-1,2,3-triazin-4(3H)-on, Smp. 184-185°C (Zers.);
7-(3-Cyanphenyl)isothiazolo[4,5-d]-1,2,3-triazin-4(3H)-on, Smp. 190°C (Zers.);

7-(3-Methoxyphenyl)isothiazolo[4,5-d]-1,2,3-triazin-4(3H)-on, Smp. 189-190°C;
7-(3-Fluorphenyl)isothiazolo[4,5-d]-1,2,3-triazin-4(3H)-on, Smp. 183°C (Zers.);
7-(3-Trifluormethylphenyl)isothiazolo[4,5-d]-1,2,3-triazin-4(3H)-on, Smp. 215-216°C (Zers.);
7-(4-Isopropoxyphenyl)isothiazolo[4,5-d]-1,2,3-triazin-4(3H)-on, Smp. 187°C (Zers.);
7-(4-Benzyloxyphenyl)isothiazolo[4,5-d]-1,2,3-triazin-4(3H)-on, Smp. 197°C (Zers.);
7-(4-Nitrophenyl)isothiazolo[4,5-d]-1,2,3-triazin-4(3H)-on, Smp. 201-204°C (Zers.);
7-(4-Chlorphenyl)isothiazolo[4,5-d]-1,2,3-triazin-4(3H)-on, Smp. 191°C (Zers.);
7-(4-Bromphenyl)isothiazolo[4,5-d]-1,2,3-triazin-4(3H)-on, Smp. 207°C (Zers.);
7-(4-Phenoxyphenyl)isothiazolo[4,5-d]-1,2,3-triazin-4(3H)-on, Smp. 170°C (Zers.);
7-(2-Naphthyl)isothiazolo[4,5-d]-1,2,3-triazin-4(3H)-on, Smp. 189°C (Zer.);
7-(2,3-Dihydro-5-benzofuranyl)isothiazolo[4,5-d]-1,2,3-triazin-4(3H)-on, Smp. 209°C (Zers.);
7-(3-Fluor-4-methoxyphenyl)isothiazolo[4,5-d]-1,2,3-triazin-4(3H)-on, Smp. 198-199°C (Zers.);
7-(3-Chlor-4-methoxyphenyl)isothiazolo[4,5-d]-1,2,3-triazin-4(3H)-on, Smp. 210°C (Zers.);
7-(3-Trifluormethyl-4-methoxyphenyl)isothiazolo[4,5-d]-1,2,3-triazin-4(3H)-on, Smp. 232°C (Zers.);
7-(3,5-Dimethylphenyl)isothiazolo[4,5-d]-1,2,3-triazin-4(3H)-on, Smp. 188°C (Zers.);
7-(4-Benzylphenyl)isothiazolo[4,5-d]-1,2,3-triazin-4(3H)-on, Smp. 174-175°C (Zers.);
7-(3-Isopropylphenyl)isothiazolo[4,5-d]-1,2,3-triazin-4(3H)-on, Smp. 155-156°C (Zers.);
7-[3,5-bis(Trifluormethyl)phenyl]isothiazolo[4,5-d]-1,2,3-triazin-4(3H)-on, Smp. 240-242°C (Zers.);
7-(3-Trifluormethyl-4-benzyloxyphenyl)isothiazolo[4,5-d]-1,2,3-triazin-4(3H)-on, Smp. 199°C (Zers.);
7-(4-Aethylphenyl)isothiazolo[4,5-d]-1,2,3-triazin-4(3H)-on, Smp. 172-173°C (Zers.);
7-(4-Isobutylphenyl)isothiazolo[4,5-d]-1,2,3-triazin-4(3H)-on, Smp. 178°C (Zers.);
7-(3-Cyan-4-methoxyphenyl)isothiazolo[4,5-d]-1,2,3-triazin-4(3H)-on, Smp. 216-219°C (Zers.);
7-(4-Allyloxy-3-cyanphenyl)isothiazolo[4,5-d]-1,2,3-triazin-4(3H)-on, Smp. 185-187°C (Zers.);
7-[3-Cyan-4-(3-hydroxypropoxy)phenyl]isothiazolo[4,5-d]-1,2,3-triazin-4(3H)-on, Smp. 194-197°C (Zers.);
und
7-(3-Cyan-4-methylthiophenyl)isothiazolo[4,5-d]-1,2,3-triazin-4(3H)-on, Smp. 204-205°C (Zers.).

Beispiel 5

22,5 g 4-Amino-3-(4-benzyloxyformamidophenyl)-5-isothiazolcarboxamid in 25 ml konz. Salzsäure und 150 ml N-Methylpyrrolidon werden in einem Eisbad gekühlt und während der Zugabe von 4,89 g Natriumnitrit in 20 ml Wasser gerührt. Nach einer halben Stunde werden 150 ml Wasser zugegeben, und der ausgefallene Niederschlag abfiltriert, mit Wasser gewaschen, im Vakuum getrocknet und aus Aethylacetat umkristalisiert, wobei man 16,6 g 7-(4-Benzyloxyformamidophenyl)isothiazolo[4,5-d]-1,2,3-triazin-4(3H)-on erhält, Smp. 190-191°C (Zers.).

Beispiel 6

1 g 7-(4-Benzyloxyformamidophenyl)isothiazolo[4,5-d] -1,2,3-triazin-4(3H)-on in 10 ml Trifluoressigsäure wird 1 Stunde zum Rückfluss erhitzt. Die Lösung wird eingedampft, und der Rückstand zwischen Aethylacetat und wässriger Natriumcarbonatlösung verteilt. Die wässrige Phase wird mit Essigsäure angesäuert, und der gebildete Niederschlag in heisses Aethylacetat extrahiert. Der organische Extrakt wird über Natriumsulfat getrocknet und eingedampft. Der Rückstand wird an Silicagel unter Verwendung von Aethylacetat/Petroläther (2:1) als Eluierungsmittel chromatographiert. Das Produkt wird aus Aethanol kristallisiert, dann in 30 ml Aethanol gelöst und mit 2 ml konz. Salzsäure versetzt, um das 7-(4-Aminophenyl)-isothiazolo[4,5-d] -1,2,3-triazin-4(3H)-on-hydrochlorid auszufällen, Smp. >280°C.

Beispiel 7

71 mg 7-(4-Aminophenyl)isothiazolo[4,5-d] -1,2,3-triazin-4(3H)-on-hydrochlorid werden zwischen Aethylacetat und wässriger Natriumbicarbonatlösung verteilt. Die organische Phase wird über Natriumsulfat getrocknet und eingedampft. Der Rückstand wird bei 100°C mit 5 ml Essigsäureanhydrid behandelt, bis

eine klare Lösung erhalten wird. Ueberschüssiges Essigsäureanhydrid wird abgedampft, und der Rückstand zuerst mit Aethylacetat und dann mit Aethylacetat/Methanol (19:1) als Eluierungsmittel an Silicagel chromatographiert. Das Produkt wird aus Essigsäure umkristallisiert, wobei man 7-(4-Acetamidophenyl)isothiazolo-[4,5-d]-1,2,3-triazin-4(3H)-on erhält, Smp. 239°C (Zers.).

Beispiel 8

Ozon wird durch eine Lösung von 50 mg 7-(4-Allyloxy-3-cyanphenyl)isothiazolo[4,5-d] -1,2,3-triazin-4-(3H)-on in 10 ml Aethanol und 2 ml Tetrahydrofuran während 20 Minuten geleitet. Danach lässt man während einer halben Stunde Stickstoff durch die Lösung leiten, gibt 49 mg Natriumborhydrid auf einmal zu und lässt das Reaktionsgemisch bei Raumtemperatur über Nacht stehen. Das Lösungsmittel wird dann eingedampft, und der Rückstand zwischen Aethylacetat und verdünnter Salzsäure verteilt. Die organische Phase wird über Natriumsulfat getrocknet und eingedampft, und der Rückstand aus Essigsäure umkristallisiert, wobei man 20 mg 7-[3-Cyan-4-(2-hydroxyäthoxy)phenyl]isothiazolo[4,5-d] -1,2,3-triazin-4(3H)-on als weissen Festkörper erhält, Smp. 190-193°C (Zers.).

Beispiel 9

Eine Lösung von 500 mg 7-(4-Allyloxy-3-cyanphenyl)isothiazolo[4,5-d] -1,2,3-triazin-4(3H)-on und 200 mg N-Methylmorpholin-N-oxid in 15 ml Dimethylformamid und 5 ml Wasser wird mit 1,75 ml einer 0,4%igen Lösung von Osmiumtetroxid in Wasser versetzt. Das Gemisch wird 7 Tage bei Raumtemperatur gerührt und dann mit einer Lösung von 220 mg Natriumdithionit in 5 ml Wasser versetzt. Das Lösungsmittel wird abgedampft, und der Rückstand mit heissem Aethylacetat extrahiert und filtriert, und das Filtrat eingedampft. Umkristallisation des Rückstands aus Essigsäure liefert 160 mg 7-[3-Cyan-4-(2,3-dihydroxypropoxy)phenyl]isothiazolo[4,5-d] -1,2,3-triazin-4(3H)-on als weissen Festkörper, Smp. 195°C (Zers.).

Beispiel 10

562 mg 3-Amino-2-(3-trifluormethylphenyl)-4-pyridincarboxamid werden bei 0°C in 5 ml konz. Salzsäure gerührt, während man 160 mg Natriumnitrit in 1 ml Wasser zugibt. Nach einer halben Stunde wird das Gemisch mit Wasser und Aethylacetat verdünnt und mit Natriumbicarbonat basisch gestellt. Die organische Phase wird abgetrennt, über Natriumsulfat getrocknet und eingedampft, und der Rückstand durch Chromatographie an Silicagel unter Verwendung von Aethylacetat/Petroläther (1:1) als Eluierungsmittel gereinigt. Dabei werden 400 mg 8-(3-Trifluormethylphenyl)pyrido[3,4-d] -1,2,3-triazin-4(3H)-on in Form eines Festkörpers erhalten, Smp. 192-193°C (Zers.).

Beispiel 11

639 mg 3-Amino-2-phenyl-4-pyridincarboxamid in 7 ml konz. Salzsäure werden in einem Eisbad während der Zugabe von 240 mg Natriumnitrit in 2 ml Wasser gerührt. Nach einer halben Stunde gibt man bis zur basischen Reaktion des Gemisches wässrige Natriumbicarbonatlösung zu und extrahiert das Produkt danach in Aethylacetat. Der Aethylacetatextrakt wird über Natriumsulfat getrocknet und eingedampft, und der Rückstand aus Aethanol umkristallisiert, wobei man 420 mg 8-Phenylpyrido[3,4-d]-1,2,3-triazin-4(3H)-on erhält, Smp. 211°C (Zers.).

Das als Ausgangsmaterial eingesetzte 3-Amino-2-phenyl-4-pyridincarboxamid wurde wie folgt hergestellt:

1 g 3-Amino-2-phenyl-4-pyridincarbonitril wird in 20 ml Wasser und 10 ml Dioxan in Gegenwart von 4 g Amberlite IRA-400-OH (basische Form) 3,5 Stunden zum Rückfluss erhitzt. Das Harz wird dann abfiltriert, und das Filtrat eingedampft. Der Rückstand wird an Silicagel mit Methanol/Aethylacetat (1:19) als Eluierungsmittel chromatographiert, und das Produkt aus Aethylacetat/Petroläther umkristallisiert, wobei man 900 mg 3-Amino-2-phenyl-4-pyridincarboxamid erhält, Smp. 163-164°C.

Die folgenden Pyridincarboxamid-Ausgangsstoffe wurden in analoger Weise hergestellt:

3-Amino-2-(3-fluor-4-methoxyphenyl)-4-pyridincarboxamid, Smp. 193-195°C;
3-Amino-2-(3-Chlor-4-methoxyphenyl)-4-pyridincarboxamid, Smp. 213-215°C;
3-Amino-2-(4-methoxy-3-trifluormethylphenyl)-4-pyridincarboxamid, Smp. 195-197°C; und
3-Amino-2-(4-benzyloxy-3-trifluormethylphenyl)-4-pyridincarboxamid, Smp. 155-157°C.

Beispiel 12

300 mg 3-Amino-2-(4-methoxyphenyl)-4-pyridincarboxamid werden bei 0°C in 5 ml Eisessig und 1 ml konz. Salzsäure gerührt, während man 128 mg Natriumnitrit in 2 ml Wasser zugibt. Man entfernt das Kühlbad, gibt 3 ml Wasser zu, rührt das Reaktionsgemisch während einer halben Stunde und filtriert das ausgefallene Produkt ab. Umkristallisation aus wässrigem Dimethylformamid liefert 275 mg 8-(4-Methoxyphenyl)pyrido[3,4-d]-1,2,3-triazin-4(3H)-on als gelbe Nadeln, Smp. 226°C (Zers.).

Die folgenden Verbindungen wurden in analoger Weise hergestellt:

8-(4-Benzyloxyphenyl)pyrido[3,4-d]-1,2,3-triazin-4(3H)-on, Smp. 221°C (Zers.);
8-(3-Fluor-4-methoxyphenyl)pyrido[3,4-d]-1,2,3-triazin-4(3H)-on, Smp. 228-230°C (Zers.);
8-(3-Chlor-4-methoxyphenyl)pyrido[3,4-d]-1,2,3-triazin-4(3H)-on, Smp. 235-237°C (Zers.);
8-(4-Methoxy-3-trifluormethylphenyl)pyrido[3,4-d]-1,2,3-triazin-4(3H)-on, Smp. 233-235°C (Zers.); und
8-(4-Benzyloxy-3-trifluormethylphenyl)pyrido[3,4-d]-1,2,3-triazin-4(3H)-on, Smp. 203-205°C (Zers.).

Das im ersten Absatz dieses Beispiels als Ausgangsmaterial eingesetzte 3-Amino-2-(4-methoxyphenyl)-4-pyridincarboxamid wurde wie folgt hergestellt:

1 g 3-Amino-2-(4-methoxyphenyl)-4-pyridincarbonitril wird während 2 Stunden in 10 ml Wasser und 10 ml Dioxan in Gegenwart von 4 g Dowex 1X4-100 (OH-Form) zum Rückfluss erhitzt. Dann wird das Harz abfiltriert, und das Filtrat zur Entfernung des Dioxans eingeengt. Der wässrige Rückstand wird mit Aethylacetat extrahiert, und die organische Phase mit Wasser und Natriumchloridlösung gewaschen, über Natriumsulfat getrocknet und eingedampft. Umkristallisation des Rückstands aus Aethylacetat/Hexan liefert 500 mg 3-Amino-2-(4-methoxyphenyl)-4-pyridincarboxamid als gelben Festkörper, Smp. 179-181°C.

Das folgende Pyridincarboxamid-Ausgangsmaterial wurde in analoger Weise hergestellt:

3-Amino-2-(4-benzyloxyphenyl)-4-pyridincarboxamid, Smp. 201-202°C.

Beispiel 13

500 mg 1-(4-Methoxyphenyl)-5-amino-4-pyrazolcarboxamid in 9 ml Eisessig und 5 ml konz. Salzsäure werden während der Zugabe einer Lösung von 150 mg Natriumnitrit in 3 ml Wasser bei 0°C gerührt. Man hält danach das Reaktionsgemisch für 10 Minuten bei 0°C, lässt es danach auf Raumtemperatur aufwärmen und rührt es weitere 20 Minuten. Das ausgefallene Produkt wird abfiltriert und aus wässrigem Dimethylformamid umkristallisiert. Dabei erhält man 150 mg 7-(4-Methoxy phenyl)-7H-pyrazolo[3,4-d]-1,2,3-triazin-4(3H)-on, Smp. 158-160°C.

Da als Ausgangsmaterial eingesetzte 1-(4-Methoxyphenyl)-5-amino-4-pyrazolcarboxamid wurde wie folgt hergestellt:

1,5 g 1-(4-Methoxyphenyl)-5-amino-4-pyrazolcarbonitril werden bei 10-15°C solange mit 10 ml konz. Schwefelsäure gerührt, bis der ganze Festkörper aufgelöst ist. Die Reaktionslösung wird danach auf 60 g zerkleinertes Eis gegossen und mit konz. Ammoniaklösung basisch gestellt. Das ausgefallene Produkt wird abfiltriert, mit Wasser gewaschen und aus Aethanol umkristallisiert, wobei man 900 mg 1-(4-Methoxyphenyl)-5-amino-4-pyrazolcarboxamid erhält, Smp. 213-215°C.

Beispiel 14

1 g 5-Amino-1-phenyl-4-pyrazolcarboxamid in 18 ml Eisessig und 10 ml konz. Salzsäure wird während der Zugabe einer Lösung von 0,4 g Natriumnitrit in 6 ml Wasser bei 0°C gerührt. Man hält danach das Gemisch 20 Minuten bei 0°C, lässt es dann auf Raumtemperatur aufwärmen und rührt es weitere 2 Stunden. Das ausgefallene Produkt wird abfiltriert und aus Aethanol umkristallisiert, wobei man 580 mg 7-Phenyl-7H-pyrazolo[3,4-d]-1,2,3-triazin-4(3H)-on erhält, Smp. 150-151°C.

Das als Ausgangsmaterial eingesetzte 5-Amino-1-phenyl-4-pyrazolcarboxamid wurde wie folgt hergestellt:

9 g 5-Amino-1-phenyl-4-pyrazolcarbonitril werden bei 10-15°C solange mit 40 ml konz. Schwefelsäure gerührt, bis sich der ganze Festkörper aufgelöst hat. Die Reaktionslösung wird dann auf 150 g zerkleinertes Eis gegossen und mit konz. Ammoniaklösung basisch gestellt. Das ausgefallene Produkt wird abfiltriert und durch Chromatographie an Silicagel unter Verwendung von 5% Methanol in Aethylacetat als Eluierungsmittel gereinigt. Auf diese Weise erhält man 5,8 g 5-Amino-1-phenyl-4-pyrazolcarboxamid. Eine aus Wasser umkristallisierte Probe schmilzt bei 163-165°C.

Beispiel 15

Die folgenden Verbindungen wurden in analoger Weisen wie in den Beispielen 13 und 14 beschrieben hergestellt:

7(3-Trifluormethylphenyl)-7H-pyrazolo[3,4-d]-1,2,3-triazin-4(3H)-on, Smp. 144-145°C;
7-(3-Chlorphenyl)-7H-pyrazolo[3,4-d]-1,2,3-triazin-4(3H)-on, Smp. 153-154°C;
7-(4-Aethylphenyl)-7H-pyrazolo[3,4-d]-1,2,3-triazin-4(3H)-on, Smp. 135-136°C;
7-(4-Methylthiophenyl)-7H-pyrazolo[3,4-d]-1,2,3-triazin-4(3H)-on, Smp. 150-152°C; und
7-(3-Chlor-4-methoxyphenyl)-7H-pyrazolo[3,4-d]-1,2,3-triazin-4(3H)-on, Smp. 161-163°C (Zers.).

Die folgenden Beispiele veranschaulichen pharmazeutische Präparate, enthaltend eine erfindungsgemässe Verbindung:

Beispiel A

Tabletten, enthaltend die folgenden Bestandteile, können in herkömmlicher Weise hergestellt werden:

| Bestandteile | Pro Tablette |
|---|---|
| 7-(3-Trifluormethyl-4-methoxyphenyl)-isothiazolo[4,5-d]-1,2,3-triazin-4(3H)-on | 5 mg |
| Lactose | 125 mg |
| Maisstärke | 65 mg |
| Talk | 4 mg |
| Magnesiumstearat | 1 mg |
| Tablettengewicht | 200 mg |

Beispiel B

Kapseln, enthaltend die folgenden Bestandteile, können in herkömmlicher Weise hergestellt werden:

| Bestandteile | Pro Kapsel |
|---|---|
| 7-(3-Chlor-4-methoxyphenyl)isothiazolo-[4,5-d]-1,2,3-triazin-4(3H)-on | 10 mg |
| Lactose | 165 mg |
| Maisstärke | 20 mg |
| Talk | 5 mg |
| Kapselfüllgewicht | 200 mg |

**Ansprüche**

1. Triazinderivate der allgemeinen Formel

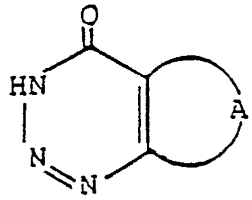

I

worin A eine Gruppe der Formel

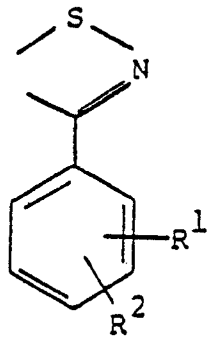

(a),

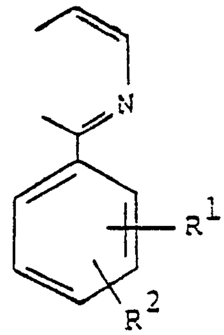

(b) oder

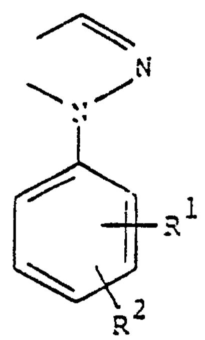

(c)

bedeutet, worin $R^1$ und $R^2$ je Wasserstoff, Halogen, Trifluormethyl, Nitro, Amino, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl($C_1$-$C_6$-alkoxy)carbonylamino oder eine Gruppe der Formel $-O-CH_2-R^3$ oder, wenn sie benachbart sind, zusammen eine Gruppe der Formel -CH = CH-CH = CH-oder $-CH_2-CH_2-O-$und $R^3$ Hydroxy-($C_1$-$C_4$-alkyl) oder vicinales Dihydroxy-($C_2$-$C_5$-alkyl) bedeuten,

und pharmazeutisch verwendbare Säureadditionssalze solcher Verbindungen der Formel 1, worin $R^1$ und/oder $R^2$ Amino bedeuten.

2. Verbindungen gemäss Anspruch 1, worin A eine Gruppe der Formel (a), (b) oder (c) bedeutet, worin $R^1$ und $R^2$ je Wasserstoff, Halogen, Trifluormethyl, Nitro, Amino, Cyan, $C_1$-$C_6$-Alkyl, $C_1$-$C_6$-Alkoxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy) oder Aryl-($C_1$-$C_6$-alkoxy)-carbonylamino oder, wenn sie benachbart sind, zusammen eine Gruppe der Formel -CH = CH-CH = CH-oder $-CH_2-CH_2-O-$bedeuten.

3. Verbindungen gemäss Anspruch 1 oder 2, worin A die Gruppe (a) bedeutet.

4. Verbindungen gemäss Anspruch 3, worin $R^1$ Wasserstoff, Halogen, Trifluormethyl oder Cyan und $R^2$ $C_1$-$C_6$-Alkoxy, Aryl-($C_1$-$C_6$-alkoxy) oder eine Gruppe der Formel $-O-CH_2-R^3$ bedeuten, worin $R^3$ vicinales Dihydroxy-($C_2$-$C_5$-alkyl) bedeutet, mit der Massgabe, dass mindestens einer von $R^1$ und $R^2$ von Wasserstoff verschieden ist.

5. 7-(3-Trifluormethyl-4-methoxyphenyl)isothiazolo[4,5-d]-1,2,3-triazin-4(3H)-on.

6. 7-(3-Chlor-4-methoxyphenyl)isothiazolo[4,5-d]-1,2,3-triazin-4(3H)-on.

7. 7-(3-Fluor-4-methoxyphenyl)isothiazolo[4,5-d]-1,2,3-triazin-4(3H)-on.

8. 7-(3-Trifluormethylphenyl)isothiazolo[4,5-d]-1,2,3-triazin-4(3H)-on.

9. 7-(4-Isopropoxyphenyl)isothiazolo[4,5-d]-1,2,3-triazin-4(3H)-on.

10. 7-(4-Benzyloxyphenyl)isothiazolo[4,5-d]-1,2,3-triazin-4(3H)-on.

11. 7-(3-Cyan-4-methoxyphenyl)isothiazolo[4,5-d]-1,2,3-triazin-4(3H)-on.

12. 7-[3-Cyan-4-(2,3-dihydroxypropoxy)phenyl]isothiazolo[4,5-d]-1,2,3-triazin-4(3H)-on.

13. Verbindungen gemäss Anspruch 1 oder 2, worin A die Gruppe (c) bedeutet.

14. Verbindungen gemäss Anspruch 13, worin $R^1$ Wasserstoff und $R^2$ $C_1$-$C_6$-Alkoxy bedeuten.

15. 7-(4-Methoxyphenyl)-7H-pyrazolo[3,4-d]-1,2,3-triazin-4(3H)-on.

16. 7-Phenylisothiazolo[4,5-d]-1,2,3-triazin-4(3H)-on,
7-(3-Aethylphenyl)isothiazolo[4,5-d]-1,2,3-triazin-4(3H)-on,
7-(3-Methylphenyl)isothiazolo[4,5-d]-1,2,3-triazin-4(3H)-on,
7-(3-Chlorphenyl)isothiazolo[4,5-d]-1,2,3-triazin-4(3H)-on,
7-(3-Bromphenyl)isothiazolo[4,5-d]-1,2,3-triazin-4(3H)-on,
7-(3-Cyanphenyl)isothiazolo[4,5-d]-1,2,3-triazin-4(3H)-on,
7-(3-Methoxyphenyl)isothiazolo[4,5-d]-1,2,3-triazin-4(3H)-on,
7-(4-Methoxyphenyl)isothiazolo[4,5-d]-1,2,3-triazin-4(3H)-on,
7-(3-Fluorphenyl)isothiazolo[4,5-d]-1,2,3-triazin-4(3H)-on,
7-(4-Acetamidophenyl)isothiazolo[4,5-d]-1,2,3-triazin-4(3H)-on,
7-(4-Benzyloxyformamidophenyl)isothiazolo[4,5-d]-1,2,3-triazin-4(3H)-on,
7-(4-Aminophenyl)isothiazolo[4,5-d]-1,2,3-triazin-4(3H)-on,
7-(4-Nitrophenyl)isothiazolo[4,5-d]-1,2,3-triazin-4(3H)-on,
7-(4-Chlorphenyl)isothiazolo[4,5-d]-1,2,3-triazin-4(3H)-on,
7-(4-Bromphenyl)isothiazolo[4,5-d]-1,2,3-triazin-4(3H)-on,
7-(4-Phenoxyphenyl)isothiazolo[4,5-d]-1,2,3-triazin-4(3H)-on,
7-(2-Naphthyl)isothiazolo[4,5-d]-1,2,3-triazin-4(3H)-on,
7-(2,3-Dihydro-5-benzofuranyl)isothiazolo[4,5-d]-1,2,3-triazin-4(3H)-on,
7-(3,5-Dimethylphenyl)isothiazolo[4,5-d]-1,2,3-triazin-4(3H)-on,
7-(4-Benzylphenyl)isothiazolo[4,5-d]-1,2,3-triazin-4(3H)-on,
7-(3-Isopropylphenyl)isothiazolo[4,5-d]-1,2,3-triazin-4(3H)-on,
7-[3,5-bis(Trifluormethyl)phenyl]isothiazolo[4,5-d]-1,2,3-triazin-4(3H)-on,
7-(3-Trifluormethyl-4-benzyloxyphenyl)isothiazolo[4,5-d]-1,2,3-triazin-4(3H)-on,
8-Phenylpyrido[3,4-d]-1,2,3-triazin-4(3H)-on,
8-(3-Trifluormethylphenyl)pyrido[3,4-d]-1,2,3-triazin-4(3H)-on,
7-Phenyl-7H-pyrazolo[3,4-d]-1,2,3-triazin-4(3H)-on,
7-(3-Trifluormethylphenyl)-7H-pyrazolo[3,4-d]-1,2,3-triazin-4(3H)-on,
7-(3-Chlorphenyl)-7H-pyrazolo[3,4-d]-1,2,3-triazin-4(3H)-on,
7-(4-Aethylphenyl)-7H-pyrazolo[3,4-d]-1,2,3-triazin-4(3H)-on,
7-(4-Methylthiophenyl)-7H-pyrazolo[3,4-d]-1,2,3-triazin-4(3H)-on und
7-(3-Chlor-4-methoxyphenyl)-7H-pyrazolo[3,4-d]-1,2,3-triazin-4(3H)-on.

17. 7-(4-Aethylphenyl)isothiazolo[4,5-d]-1,2,3-triazin-4(3H)-on,
7-(4-Isobutylphenyl)isothiazolo[4,5-d]-1,2,3-triazin-4(3H)-on,
7-(4-Allyloxy-3-cyanphenyl)isothiazolo[4,5-d]-1,2,3-triazin-4(3H)-on,
7-[3-Cyan-4-(2-hydroxyäthoxy)phenyl]isothiazolo[4,5-d]-1,2,3-triazin-4(3H)-on,
7-[3-Cyan-4-(3-hydroxypropoxy)phenyl]isothiazolo[4,5-d]-1,2,3-triazin-4(3H)-on,
7-[3-Cyan-4-methylthiophenyl]isothiazolo[4,5-d]-1,2,3-triazin-4(3H)-on,
8-(4-Methoxyphenyl)pyrido[3,4-d]-1,2,3-triazin-4(3H)-on,
8-(4-Benzyloxyphenyl)pyrido[3,4-d]-1,2,3-triazin-4(3H)-on,
8-(3-Fluor-4-methoxyphenyl)pyrido[3,4-d]-1,2,3-triazin-4(3H)-on,
8-(3-Chlor-4-methoxyphenyl)pyrido[3,4-d]-1,2,3-triazin-4(3H)-on,
8-(4-Methoxy-3-trifluormethylphenyl)pyrido[3,4-d]-1,2,3-triazin-4(3H)-on und
8-(4-Benzyloxy-3-trifluormethylphenyl)pyrido[3,4-d]-1,2,3-triazin-4(3H)-on.

18. Triazinderivat gemäss einem der Ansprüche 1-17 zur Anwendung als therapeutischer Wirkstoff.

19. Triazinderivat gemäss einem der Ansprüche 1-17 zur Anwendung bei der Bekämpfung bzw. Verhütung von Ischämie oder Gicht.

20. Verfahren zur Herstellung einer Verbindung gemäss einem der Ansprüche 1-17, dadurch gekennzeichnet, dass man

a) zur Herstellung einer Verbindung der Formel I, worin $R^1$ und $R^2$ je Wasserstoff, Halogen, Trifluormethyl, Nitro, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^3$ oder, wenn sie benachbart sind, zusammen eine Gruppe der Formel $-CH=CH-CH=CH-$ oder $-CH_2-CH_2-O-$ und $R^3$ Hydroxy-($C_1$-$C_4$-alkyl) bedeuten, eine Verbindung der allgemeinen Formel

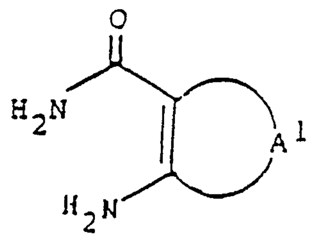

II

worin $A^1$ eine Gruppe der Formel

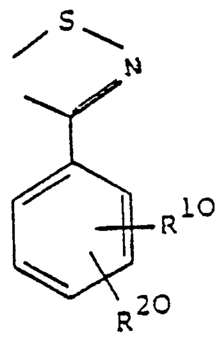

(a'),

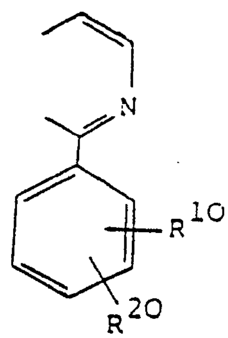

(b') oder

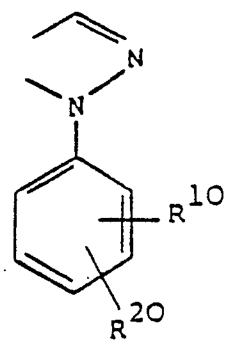

(c')

bedeutet, worin $R^{10}$ und $R^{20}$ je Wasserstoff, Halogen, Trifluormethyl, Nitro, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^{30}$ oder, wenn sie benachbart sind, zusammen eine Gruppe der Formel $-CH=CH-CH=CH-$oder $-CH_2-CH_2-O-$und $R^{30}$ Hydroxy-($C_1$-$C_4$-alkyl) bedeuten,

mit salpetriger Säure umsetzt, oder

b) zur Herstellung einer Verbindung der Formel I, worin $R^1$ und/oder $R^2$ Amino bedeuten, die Aryl-($C_1$-$C_6$-alkoxy)carbonylaminogruppe(n) in einer Verbindung der Formel I, worin $R^1$ und/oder $R^2$ Aryl-($C_1$-$C_6$-alkoxy)carbonylamino bedeutet, spaltet, oder

c) zur Herstellung einer Verbindung der Formel I, worin $R^1$ und/oder $R^2$ $C_1$-$C_6$-Alkanoylamino bedeuten, eine Verbindung der Formel I, worin $R^1$ und/oder $R^2$ Amino bedeuten, entsprechend acyliert, oder

d) zur Herstellung einer Verbindung der Formel I, worin $R^1$ und/oder $R^2$ eine Gruppe der Formel $-O-CH_2-R^3$ bedeuten, worin $R^3$ Hydroxy-($C_1$-$C_4$-alkyl) bedeutet, eine Verbindung der Formel I, worin $R^1$ und/oder $R^2$ $C_3$-$C_6$-Alkenyloxy bedeuten, mit Ozon umsetzt und das Reaktionsprodukt mit einem komplexen Metallhydrid reduziert, oder

e) zur Herstellung einer Verbindung der Formel I, worin $R^1$ und/oder $R^2$ einer Gruppe der Formel $-O-CH_2-R^3$ bedeuten, worin $R^3$ vicinales Dihydroxy-($C_2$-$C_5$-alkyl) bedeutet, eine Verbindung der Formel I, worin $R^1$ und/oder $R^2$ $C_3$-$C_6$-Alkenyloxy bedeuten, mit Osmiumtetroxid umsetzt, und

f) erwünschtenfalls, eine erhaltene Verbindung der Formel I, worin $R^1$ und/oder $R^2$ Amino bedeuten, in ein pharmazeutisch verwendbares Säureadditionssalz überführt.

21. Arzneimittel, enthaltend ein Triazinderivat gemäss einem der Ansprüche 1-17 und ein therapeutisch inertes Excipiens.

22. Medikament bei der Bekämpfung bzw. Verhütung von Ischämie oder Gicht, enthaltend ein Triazinderivat gemäss einem der Ansprüche 1-17 und ein therapeutisch inertes Excipiens.

23. Verwendung eines Triazinderivates gemäss einem der Ansprüche 1-17 zur Herstellung eines Medikamentes gegen Ischämie und/oder Gicht.

Patentansprüche für folgende Vertragsstaaten : ES ; GR

1. Verfahren zur Herstellung von Triazinderivaten der allgemeinen Formel

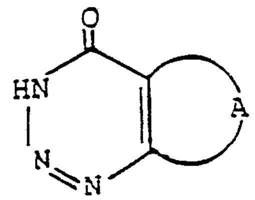

I

worin A eine Gruppe der Formel

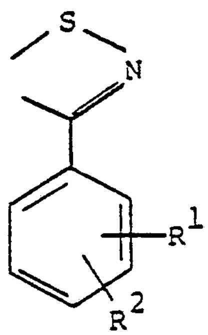

(a),

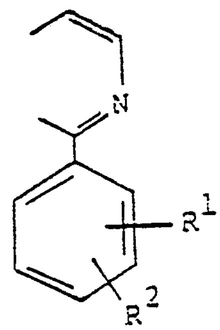

(b) oder

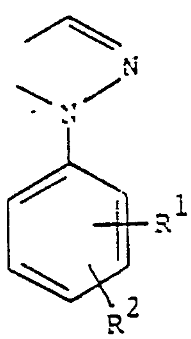

(c)

bedeutet, worin $R^1$ und $R^2$ je Wasserstoff, Halogen, Trifluormethyl, Nitro, Amino, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^3$ oder, wenn sie benachbart sind, zusammen eine Gruppe der Formel $-CH=CH-CH=CH-$oder $-CH_2-CH_2-O-$und $R^3$ Hydroxy-($C_1$-$C_4$-alkyl) oder vicinales Dihydroxy-($C_2$-$C_5$-alkyl) bedeuten,

und pharmazeutisch verwendbaren Säureadditionssalzen solcher Verbindungen der Formel I, worin $R^1$ und/oder $R^2$ Amino bedeuten, dadurch gekennzeichnet, dass man

a) zur Herstellung einer Verbindung der Formel I, worin $R^1$ und $R^2$ je Wasserstoff, Halogen, Trifluormethyl, Nitro, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel $-O-CH_2-R^3$ oder, wenn sie benachbart sind, zusammen eine Gruppe der Formel $-CH=CH-CH=CH-$oder $-CH_2-CH_2-O-$und $R^3$ Hydroxy-($C_1$-$C_4$-alkyl) bedeuten, eine Verbindung der allgemeinen Formel

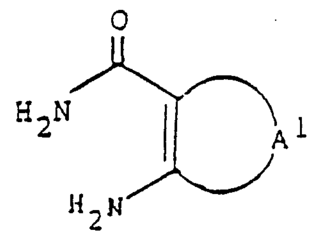

II

worin $A^1$ eine Gruppe der Formel

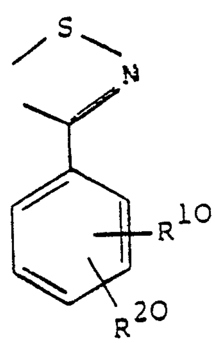

(a'),

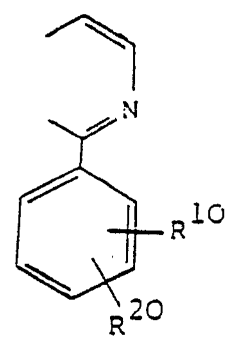

(b') oder

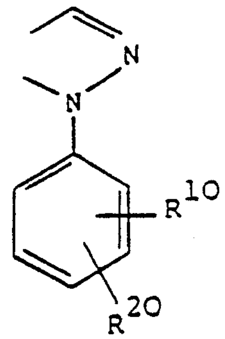

(c')

bedeutet, worin $R^{10}$ und $R^{20}$ je Wasserstoff, Halogen, Trifluormethyl, Nitro, Cyan, $C_1$-$C_6$-Alkyl, $C_1$-$C_6$-Alkoxy, $C_3$-$C_6$-Alkenyloxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy), Aryl-($C_1$-$C_6$-alkoxy)carbonylamino oder eine Gruppe der Formel -O-$CH_2$-$R^{30}$ oder, wenn sie benachbart sind, zusammen eine Gruppe der Formel -CH = CH-CH = CH-oder -$CH_2$-$CH_2$-O-und $R^{30}$ Hydroxy-($C_1$-$C_4$-alkyl) bedeuten,

mit salpetriger Säure umsetzt, oder

b) zur Herstellung einer Verbindung der Formel I, worin $R^1$ und/oder $R^2$ Amino bedeuten, die Aryl-($C_1$-$C_6$-alkoxy)carbonylaminogruppe(n) in einer Verbindung der Formel I, worin $R^1$ und/oder $R^2$ Aryl-($C_1$-$C_6$-alkoxy)carbonylamino bedeutet, spaltet, oder

c) zur Herstellung einer Verbindung der Formel I, worin $R^1$ und/oder $R^2$ $C_1$-$C_6$-Alkanoylamino bedeuten, eine Verbindung der Formel I, worin $R^1$ und/oder $R^2$ Amino bedeuten, entsprechend acyliert, oder

d) zur Herstellung einer Verbindung der Formel I, worin $R^1$ und/oder $R^2$ eine Gruppe der Formel -O-$CH_2$-$R^3$ bedeuten, worin $R^3$ Hydroxy-($C_1$-$C_4$-alkyl) bedeutet, eine Verbindung der Formel I, worin $R^1$ und/oder $R^2$ $C_3$-$C_6$-Alkenyloxy bedeuten, mit Ozon umsetzt und das Reaktionsprodukt mit einem komplexen Metallhydrid reduziert, oder

e) zur Herstellung einer Verbindung der Formel I, worin $R^1$ und/oder $R^2$ eine Gruppe der Formel -O-$CH_2$-$R^3$ bedeuten, worin $R^3$ vicinales Dihydroxy-($C_2$-$C_5$-alkyl) bedeutet, eine Verbindung der Formel I, worin $R^1$ und/oder $R^2$ $C_3$-$C_6$-Alkenyloxy bedeuten, mit Osmiumtetroxid umsetzt, und

f) erwünschtenfalls, eine erhaltene Verbindung der Formel I, worin $R^1$ und/oder $R^2$ Amino bedeuten, in ein pharmazeutisch verwendbares Säureadditionssalz überführt.

2. Verfahren gemäss Anspruch 1, worin A eine Gruppe der Formel (a), (b) oder (c) bedeutet, worin $R^1$ und $R^2$ je Wasserstoff, Halogen, Trifluormethyl, Nitro, Amino, Cyan, $C_1$-$C_6$-Alkyl, $C_1$-$C_6$-Alkoxy, $C_1$-$C_6$-Alkylthio, $C_1$-$C_6$-Alkanoylamino, Aryloxy, Aryl-($C_1$-$C_6$-alkyl), Aryl-($C_1$-$C_6$-alkoxy) oder Aryl-($C_1$-$C_6$-alkoxy)-carbonylamino oder, wenn sie benachbart sind, zusammen eine Gruppe der Formel -CH = CH-CH = CH-oder $-CH_2-CH_2-O-$bedeuten.

3. Verfahren gemäss Anspruch 1 oder 2, worin A die Gruppe (a) bedeutet.

4. Verfahren gemäss Anspruch 3, worin $R^1$ Wasserstoff, Halogen, Trifluormethyl oder Cyan und $R^2$ $C_1$-$C_6$-Alkoxy, Aryl-($C_1$-$C_6$-alkoxy) oder eine Gruppe der Formel $-O-CH_2-R^3$ bedeuten, worin $R^3$ vicinales Dihydroxy-($C_2$-$C_5$-Alkyl) bedeutet, mit der Massgabe, dass mindestens einer von $R^1$ und $R^2$ von Wasserstoff verschieden ist.

5. Verfahren gemäss Anspruch 1, dadurch gekennzeichnet, dass man 7-(3-Trifluormethyl-4-methoxyphenyl)isothiazolo[4,5-d]-1,2,3-triazin-4(3H)-on herstellt.

6. Verfahren gemäss Anspruch 1, dadurch gekennzeichnet, dass man 7-(3-Chlor-4-methoxyphenyl)-isothiazolo[4,5-d]-1,2,3-triazin-4(3H)-on herstellt.

7. Verfahren gemäss Anspruch 1, dadurch gekennzeichnet, dass man 7-(3-Fluor-4-methoxyphenyl)-isothiazolo[4,5-d]-1,2,3-triazin-4(3H)-on herstellt.

8. Verfahren gemäss Anspruch 1, dadurch gekennzeichnet, dass man 7-(3-Trifluormethylphenyl)-isothiazolo[4,5-d]-1,2,3-triazin-4(3H)-on herstellt.

9. Verfahren gemäss Anspruch 1, dadurch gekennzeichnet, dass man 7-(4-Isopropoxyphenyl)-isothiazolo[4,5-d]-1,2,3-triazin-4(3H)-on herstellt.

10. Verfahren gemäss Anspruch 1, dadurch gekennzeichnet, dass man 7-(4-Benzyloxyphenyl)-isothiazolo[4,5-d]-1,2,3-triazin-4(3H)-on herstellt.

11. Verfahren gemäss Anspruch 1, dadurch gekennzeichnet, dass man 7-(3-Cyan-4-methoxyphenyl)-isothiazolo[4,5-d]-1,2,3-triazin-4(3H)-on herstellt.

12. Verfahren gemäss Anspruch 1, dadurch gekennzeichnet, dass man 7-[3-Cyan-4-(2,3-dihydroxypropoxy)phenyl]isothiazolo[4,5-d]-1,2,3-triazin-4(3H)-on herstellt.

13. Verfahren gemäss Anspruch 1 oder 2, worin A die Gruppe (c) bedeutet.

14. Verfahren gemäss Anspruch 13, worin $R^1$ Wasserstoff und $R^2$ $C_1$-$C_6$-Alkoxy bedeuten.

15. Verfahren gemäss Anspruch 1, dadurch gekennzeichnet, dass man 7-(4-Methoxyphenyl)-7H-pyrazolo[3,4-d]-1,2,3-triazin-4(3H)-on herstellt.